# EUROPEAN PATENT SPECIFICATION

(11) **EP 1 958 880 B1**
(45) Date of publication and mention of the grant of the patent: **11.08.2010**
(21) Application number: 08150867.3
(22) Date of filing: 31.01.2008
(51) Int. Cl.: B65C 3/16, B65C 9/06, A61M 5/00, B65D 25/56

(54) **Disposable container and method for labelling it.**
Einwegbehälter und Verfahren zu dessen Etikettierung
Récipient jetable et procédé pour l'étiqueter

(30) Priority: 14.02.2007 IT BO20070079
(43) Date of publication of application: 20.08.2008
(73) Proprietor: CO.RI.M.A. S.r.l., 53035 Monteriggioni (SI) (IT)
(72) Inventor: Anatrini, Dario, 53100 Siena (IT)
(74) Representative: Lanzoni, Luciano

(56) References cited:
- WO-A-02/11787
- US-A- 3 653 176
- US-A- 3 791 009
- US-A- 3 835 897

## Description

The present invention relates to a disposable container, a disposable syringe, and a method for labelling said container.

As is known, disposable syringes are filled with the relative medicine during their assembly, in a sterile environment.

The transparent body, usually cylindrical, of each syringe normally bears graduated marks, which may be stamped and/or moulded on it, to allow, for example, partial use of the medicine according to the dose required for the patient.

In many cases, in addition to the graduated marks, the syringe body must also bear a label relative to the medicine it contains. In such cases, to prevent the label from covering and hiding the graduated marks on the syringe, the width of the label is less than the outer extension of the syringe body. In this way, the label can be wrapped around the syringe body except for the longitudinal band of the body bearing the graduated marks.

An example of a disposable syringe can bee seen in document WO 02/11787.

This document discloses a capped syringe that has been labeled and filled. A cap covers and protects the sterility of the dispensing luer tip.

Since the barrel of the syringe is full, the plunger is extended longitudinally. A flap or substrate for a label is provided by two strips of adhesive tape both of which are wrapped around and adhered to respectively opposite sides of the barrel and adhered to each other in face-to-face relation in extensions of the adhesive tape that extend in diametrically opposite directions from the barrel. At least one of the adhesive tapes is transparent so that the graduation marks that are on most conventional syringes can be seen through the adhesive tape.

However, in some cases, in particular in the case of syringes with a small cross-section, the width set for the label may be so low that the most common automatic labelling machines have difficulty applying the label.

Attempts were made to solve the problem by using labels themselves bearing the graduated marks. That allows the width of the label to be substantially equal to or even slightly greater than the outer extension of the syringe body. However, the graduated marks were not very reliable because they were affected by possible errors, although small, in the application of the label.

The aim of the present invention is to provide a container, a disposable syringe and a method for labelling it according to which the label can easily be applied without compromising the legibility of the graduated marks on the syringe.

Accordingly, the present invention provides a disposible container, a disposable syringe and a method for labelling it, as described in the claims herein.

The present invention is now described, by way of example and without limiting the scope of application, with reference to the accompanying drawings, in which:
- Figures 1 and 5 illustrate a disposable syringe with a respective label, made and applied in accordance with the present invention;
- Figure 2 illustrates the label of Figure 1 laid out flat;
- Figure 3 is a perspective view with some parts cut away for clarity of a labelling machine able to implement the method in accordance with the present invention;
- Figure 4 is a perspective view with some parts cut away for clarity of a detail of Figure 3;
- Figure 6 illustrates an alternative embodiment of the label of Figure 2, laid out flat.

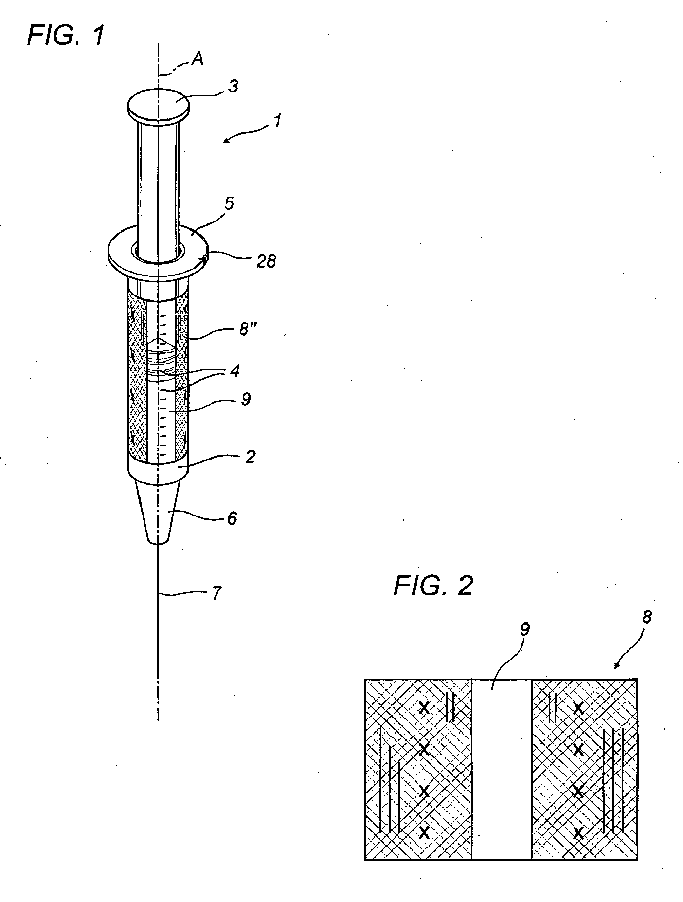

With reference to Figure 1, the numeral 1 denotes, as a non-limiting example of a disposable container, a disposable syringe comprising a transparent tubular body 2, containing a medicine, and a plunger 3, slidably connected in a sealed fashion to the body 2 for dispensing the medicine.

The body 2 is cylindrical and has a central longitudinal axis A, along which the plunger 3 slides. At a longitudinal band, the body 2 bears, printed and/or moulded, graduated marks 4, whose function is to allow partial use of the medicine, according to the dose required for the patient.

At the longitudinal end from which the plunger 3 comes out, the body 2 has a grip collar 5, whilst at the opposite longitudinal end the body 2 is closed down into a central through-hole by a housing 6 having the shape of a truncated cone, a needle 7 engaging coaxially in the housing 6.

Advantageously, wrapped around the body 2 but not necessarily through 360°, about the axis A, there is an adhesive label 8 describing the medicine. The special feature of this label is that it has a transparent portion 9 placed over the graduated marks 4. In particular, as Figure 2 more clearly shows, the transparent portion 9 is a longitudinal band, substantially central and rectangular, of the label 8.

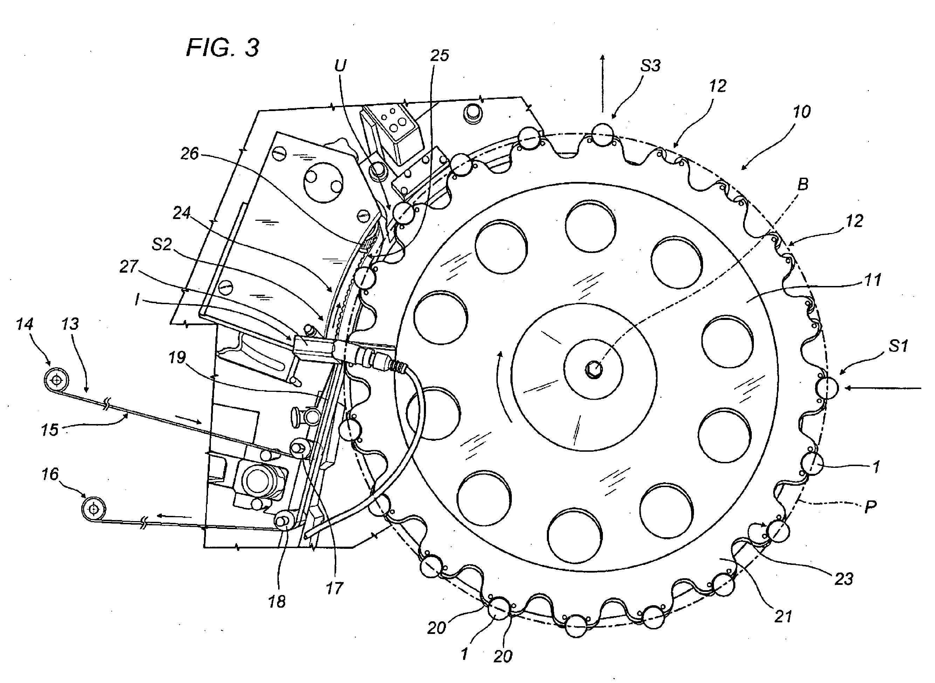

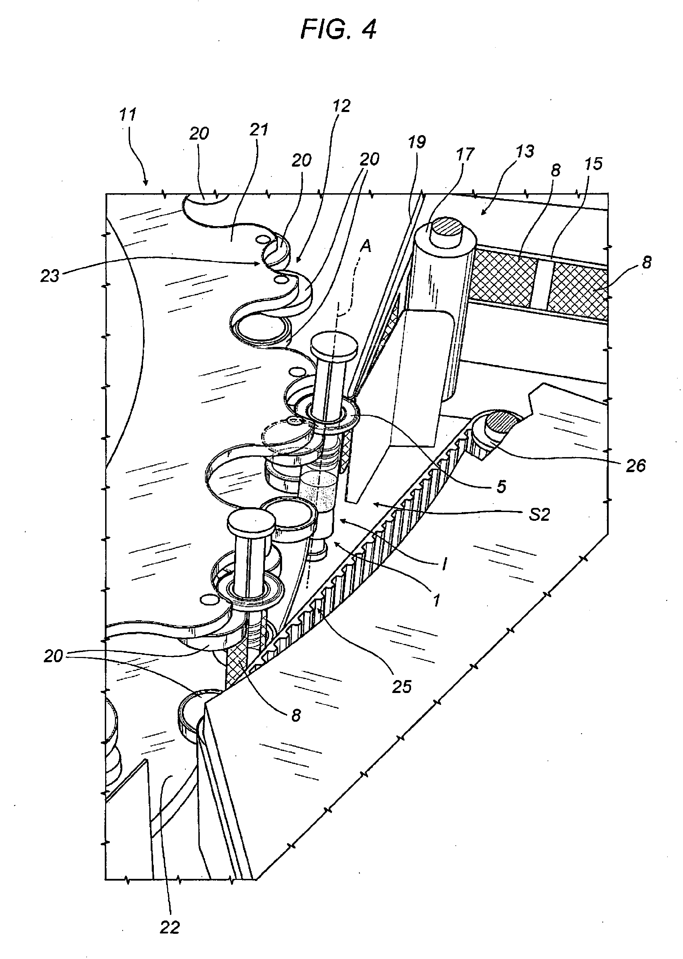

The label 8 is applied on the body 2 using the packing machine illustrated in Figures 3 and 4, labelled 10 as a whole.

The machine 10 comprises a carrousel 11 which rotates with a stepping motion about a vertical central axis B, clockwise in Figure 3.

The carrousel has a plurality of outer cavities 12, evenly distributed about the axis B and each designed to convey a syringe 1, with its axis A vertical and with the needle 7 pointing downwards, on a circular path P.

The path P extends between a loading station S1, in which the syringes 1 are inserted in the respective cavities 12, and an unloading station S3, where each syringe 1, after being checked, is taken out of the respective cavity 12 and directed towards a rejection path or an acceptance path.

Inserted between the stations S1 and S3 there is a labelling station S2, where each syringe 1 receives its label 8 and along which each syringe 1 has the label 8 wrapped around it like a tube.

Converging at the station S2 infeed I there is a line 13 designed to feed an orderly succession of labels 8. The line 13 comprises a device 14 for unwinding a continuous web 15 carrying labels 8, a device 16 for recovering the web 15, after the web, having passed through the station S2 infeed I, no longer has labels 8 on it, a first web 15 return roller 17, positioned between the device 14 and the station S2, a second web 15 return roller 18, positioned between the station S2 and the device 16, and a plate 19, around which, at the station S2 infeed I, the web 15 is wound, being suddenly bent in an L shape, so that the labels 8 peel off one after another.

As Figure 4 shows in detail, each cavity 12 supports its syringe 1 laterally with four rollers 20 having a vertical axis, coaxial with one another in pairs, so that each syringe 1 is free to rotate about its own axis A at and along the station S2. Of the four rollers 20, the two upper rollers 20 are rotatably and idly mounted on an annular plate 21, coaxial with the axis B, of the carrousel 11, whilst the other two, lower rollers 20 are rotatably and idly mounted on an annular plate 22, opposite the plate 21 in such a way that it mirrors it and also coaxial with the axis B and forming part of the carrousel 11.

The upper rollers 20 and the lower rollers 20 are mounted on the plate 21 and, respectively, on the plate 22 at the edges of respective C-shaped outer grooves 23 of the plates 21 and 22.

Relative to each cavity 12, the upper bases of the upper rollers 20 support the bottom of the collar 5 of the respective syringe 1, which is suspended in the cavity 12.

The above-mentioned infeed I is more precisely the infeed of a rolling channel 24, designed to wrap the body 2 of each syringe 1 in the respective label 8 by making the syringe 1 rotate about its own longitudinal axis A after a longitudinal edge of the label 8 has been brought into contact with the body 2 by the line 13.

On one side the channel 24 is formed by the outside of the carrousel 11 with its idle rollers 20, and on the other side by a motor-driven belt 25, opposite the outside of the carrousel 11 at a constant distance, along an arc of the carrousel.

The belt 25 is looped around at least two pulleys 26 whose axes are parallel with the axis B and rotates anti-clockwise as illustrated in Figure 3.

At the station S2 and channel 24 infeed I, and precisely above the infeed I, there is a sensor 27 designed to detect the angular position of each syringe 1 so as to allow, as is explained below, synchronisation of each syringe 1 with the respective label 8 so that the above-mentioned transparent portion 9 is placed over the graduated marks 4 on the body 2.

During machine 10 operation the syringes 1 are fed, each in its cavity 12, one after another in an orderly fashion and with a stepping motion from the station S1 towards the station S2, precisely towards the infeed I of the station S2 and the rolling channel 24.

With the carrousel 11 paused, the syringe 1 at the infeed I is synchronised with the label 8 which the line 13 has, in the meantime, stopped in a predetermined position at the station S2 infeed I. Said synchronisation, whose function is to ensure that subsequently the transparent portion 9 is precisely placed over the graduated marks 4, is carried out by rotating the syringe 1 at the infeed I at a constant speed about its own longitudinal axis A by means of the belt 25, using the sensor 27 to detect a marking 28 present on the syringe 1 collar 5, and calculating the time interval, from the moment of detection, for which the syringe 1 must be kept rotating, at said constant speed, for it to reach a predetermined angular position. Then, at the end of said time interval, after which the syringe 1 is in any case kept rotating, in a synchronised fashion the line 13 launches the label 8 in such a way that a longitudinal edge of the label makes contact with the syringe body 2. After the belt 25 has rotated the syringe 1 about its axis A until the label 8 is fully wrapped around the syringe, the belt 25 is stopped and the carrousel 11 moves forward one step.

Synchronisation of the subsequent syringe 1 causes the syringe 1 which already received the label 8 to roll again about its axis A in the channel 24.

According to alternative embodiments of this method, available to average technicians in the sector, as an alternative to the syringe 1 pause step, the syringe 1 may be kept moving and the sensor 27 can be oscillated or in any event moved over a predetermined stretch to follow syringe 1 feed, thus interacting with the latter.

Only after leaving the outfeed U of the channel 24 and the station S2 , the labelled syringes 1 are taken from the carrousel 11 at the station S3.

According to an alternative embodiment, the belt 25 is continuously kept operating at a constant speed, as the carrousel 11 is gradually moved with a stepping motion, preferably so that the syringes 1 are rolled from the infeed I to the outfeed U of the channel 24 and the station S2.

According to another alternative embodiment, the sensor 27 is substituted by a camera 27. In this case, detection of the marking 28 is substituted with detection of an image of the syringe 1 coinciding with a sample image.

In this description the term graduated marks refers to a particular type of sign or indication reproduced on the particular type of disposable container represented by the syringe. However, for the purposes of this invention, the term graduated marks shall be considered non-limiting, such that it also comprises any text, logo, trade-mark or the like reproduced on the surface of a disposable container, whether it is a syringe, a vial or other type of disposable container.

As already indicated, this description refers to a disposable syringe as a non-limiting example of a disposable container. In an equivalent way, the container could be a vial, an ampoule or the like, such as the cartridges for medicines used in the dental sector.

It shall be understood that equivalent to the solution in this description and claims, but not forming part of this invention, is the application of a label which, although without any transparent portion, is wrapped around the container for an angle of less than 360°, such that it leaves uncovered the zone of the container where the graduated marks are located.

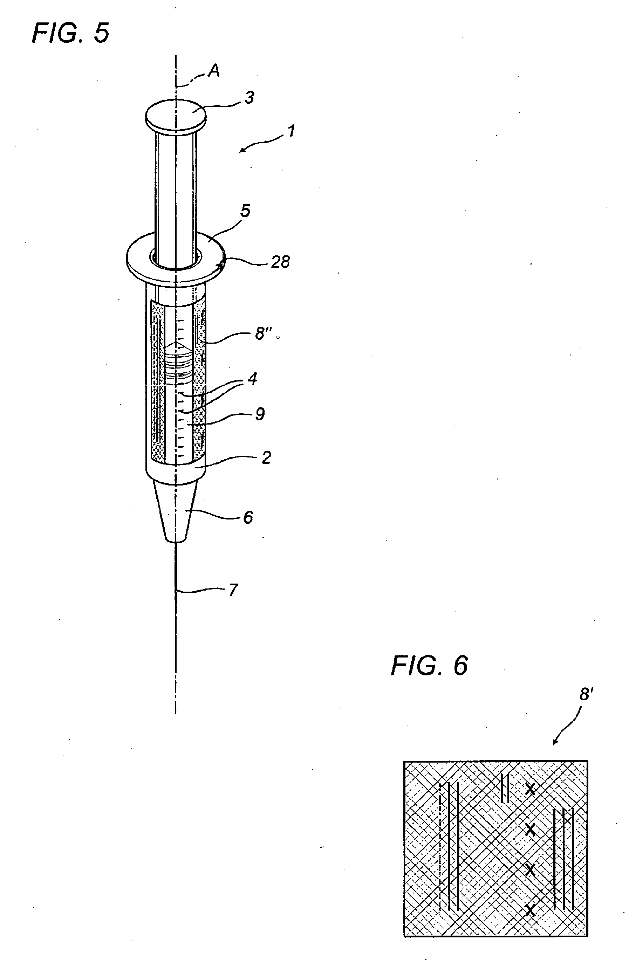

In other words, instead of using a label having a transparent portion intended to be placed over the graduated marks on the container, a label may be applied whose angular extension is less than that of the container circumference, such that the label does not cover the graduated marks and the latter are therefore legible even after application of the label. A label 8' without a transparent window and suitable for this type of application is illustrated in Figure 6. The label 8', represented in the same scale as the label 8 of Figure 2, has a reduced longitudinal extension so that it is wrapped around the syringe 1 through an angle of less than 360° and, using the method disclosed, leaves the graduated marks 4 on the syringe 1 uncovered and legible.

In contrast, Figure 5 illustrates application of another alternative embodiment of a label, indicated by the reference character 8" . Although it has a transparent window 9, this label is wrapped around the syringe 1 through an angle of less than 360°, therefore leaving a predetermined stretch of the surface of the syringe 1 uncovered.

Obviously, the invention described above achieves the preset aims and it may be modified and adapted in several ways without thereby departing from the scope of the invention indicated in the claims herein.

## Claims

1. A method for labelling disposable containers, in particular disposable syringes, comprising the step of feeding an orderly succession of containers (1) having graduated marks (4) on the body (2) towards a labelling station (S2), an orderly succession of adhesive labels (8) is fed towards the labelling station (S2), each label having a transparent portion (9); **characterised in that** in the labelling station (S2) wrapping the respective label (8) fully around the body (2) of each container (1), first bringing a longitudinal edge of the label (8) into contact with the container (1) body (2) and then rotating the container (1) about its own longitudinal axis (A); the wrapping step being preceded by synchronisation of the container (1) and the label (8) so that the transparent portion (9) is placed over the graduated marks (4).

2. The method according to claim 1, **characterised in that** synchronisation is achieved by making the container (1) rotate at a constant speed about its own longitudinal axis (A), using a sensor (27) to detect a marking (28) on the container (1), calculating the time interval, from the moment of detection, for which the container (1) must be kept rotating, at said constant speed, for it to reach a predetermined angular position.

3. The method according to claim 1, **characterised in that** synchronisation is achieved by making the container (1) rotate at a constant speed about its own longitudinal axis (A), using a camera (27) to detect an image of the container (1) coinciding with a sample image, calculating the time interval, from the moment of detection, for which the container (1) must be kept rotating, at said constant speed, for it to reach a predetermined angular position.

4. The method according to any of the claims from 1 to 3, wherein the transparent portion (9) is a longitudinal band of the label (8).

5. The method according to claim 4, wherein the longitudinal band is substantially rectangular.

6. A disposable container comprising a transparent body (2) bearing graduated marks (4) and having a label (8), **characterised in that** the label is fully wrapped around the body (2) and has a transparent portion (9) placed over the graduated marks (4) and a non-transparent portion wrapped around the transparent body (2).

7. A disposable syringe comprising a transparent body (2) containing a medicine, and a plunger (3) slidably connected to the body (2) in a sealed fashion for dispensing the medicine, the body (2) having graduated marks (4) and having a label (8; 8") describing the medicine wrapped around it, the syringe (1) being **characterised in that** the label (8; 8") is fully wrapped around the body (2) and has a transparent portion (9) placed over the graduated marks (4) and a non-transparent portion wrapped around the transparent body (2).

8. The syringe according to claim 7, **characterised in that** the transparent portion (9) is a longitudinal band of the label (8, 8").

9. The syringe according to claim 8, **characterised in that** the longitudinal band is substantially rectangular.

10. The syringe according to any of the claims from 7 to 9, **characterised in that** the label (8) is wrapped around the syringe (1) body (2) through 360°.

## Patentansprüche

1. Verfahren zur Etikettierung von Einwegbehältern, insbesondere Einwegspritzen, beinhaltend den Schritt des Zuführens einer geordneten Aufeinanderfolge von Behältern (1) mit Skalenmarkierungen (4) auf dem Körper (2) zu einer Etikettierstation (S2), während eine geordnete Aufeinanderfolge von Klebeetiketten (8), von denen jedes Etikett einen transparenten Abschnitt (9) aufweist, der Etikettierstation (S2) zugeführt wird; wobei das Verfahren **dadurch gekennzeichnet ist, dass** es vorsieht, in der Etikettierstation (S2) das entsprechende Etikett (8) vollständig um den Körper (2) jedes Behälters (1) zu hüllen, indem zuerst eine Längskante des Etiketts (8) in Berührung mit dem Körper (2) des Behälters (1) gebracht wird und dann der Behälter (1) um seine Längsachse (A) gedreht wird; wobei dem Umhüllungsschritt eine Synchronisierung des Behälters (1) und des Etiketts (8) vorausgeht, damit der transparente Abschnitt (9) über den Skalenmarkierungen (4) zu liegen kommt.

2. Verfahren nach Anspruch 1, **dadurch gekennzeichnet, dass** die Synchronisierung erreicht wird, indem der Behälter (1) veranlasst wird, mit konstanter Geschwindigkeit um seine Längsachse (A) zu drehen, ein Sensor (27) verwendet wird, um eine Markierung (28) auf dem Behälter (1) zu erfassen, und das Zeitintervall berechnet wird, für das, ab dem Moment der Erfassung, der Behälter (1) weiter mit der konstanten Geschwindigkeit gedreht werden muss, bis er eine vorbestimmte Winkelposition erreicht.

3. Verfahren nach Anspruch 1, **dadurch gekennzeichnet, dass** die Synchronisierung erreicht wird, indem der Behälter (1) veranlasst wird, mit konstanter Geschwindigkeit um seine Längsachse (A) zu drehen, eine Kamera (27) verwendet wird, um ein Bild des Behälters (1) zu erfassen, das einem Musterbild entspricht, und das Zeitintervall berechnet wird, für das, ab dem Moment der Erfassung, der Behälter (1) weiter mit der konstanten Geschwindigkeit gedreht werden muss, bis er eine vorbestimmte Winkelposition erreicht.

4. Verfahren nach einem der Ansprüche von 1 bis 3, worin der transparente Abschnitt (9) ein Längsstreifen des Etiketts (8) ist.

5. Verfahren nach Anspruch 4, worin der Längsstreifen im Wesentlichen rechteckig ist.

6. Einwegbehälter, der einen transparenten Körper (2) beinhaltet, der Skalenmarkierungen (4) trägt und ein Etikett (8) aufweist, **dadurch gekennzeichnet, dass** das Etikett vollständig um den Körper (2) gehüllt ist und einen transparenten Abschnitt (9) aufweist, der über den Skalenmarkierungen (4) angeordnet ist, und einen nichttransparenten Abschnitt, der um den transparenten Körper (2) gehüllt ist.

7. Einwegspritze, die einen transparenten Körper (2) beinhaltet, der ein Arzneimittel enthält, sowie einen Kolben (3), der gleitbar mit dem Körper (2) in abgedichteter Weise verbunden ist, um das Arzneimittel auszugeben, wobei der Körper (2) Skalenmarkierungen (4) aufweist und mit einem Etikett (8; 8"), das eine Beschreibung des enthaltenen Arzneimittels trägt, umhüllt ist, wobei die Spritze (1) **dadurch gekennzeichnet ist, dass** das Etikett (8; 8") vollständig um den Körper (2) gehüllt ist und einen transparenten Abschnitt (9) aufweist, der über den Skalenmarkierungen (4) angeordnet ist, und einen nichttransparenten Abschnitt, der um den transparenten Körper (2) gehüllt ist.

8. Spritze nach Anspruch 7, **dadurch gekennzeichnet, dass** der transparente Abschnitt (9) ein Längsstreifen des Etiketts (8, 8") ist.

9. Spritze nach Anspruch 8, worin der Längsstreifen im Wesentlichen rechteckig ist.

10. Spritze nach einem der Ansprüche von 7 bis 9, **dadurch gekennzeichnet, dass** das Etikett (8) über 360° um den Körper (2) der Spritze (1) gehüllt ist.

## Revendications

1. Un procédé pour étiqueter des récipients jetables, en particulier des seringues jetables, comprenant la phase consistant à acheminer une succession ordonnée de récipients (1), portant des repères gradués (4) sur leur corps (2), vers une station d'étiquetage (S2), une succession ordonnée d'étiquettes (8) adhésives étant alimentée vers ladite station d'étiquetage (S2), chaque étiquette présentant une partie transparente (9) ; ledit procédé étant **caractérisé en ce qu'**il prévoit, dans la station d'étiquetage (S2), l'enveloppement de l'étiquette (8) respective tout autour du corps (2) de chaque récipient (1), en amenant d'abord un bord longitudinal de l'étiquette (8) au contact du corps (2) du récipient (1) puis en faisant tournant le récipient (1) autour de son propre axe longitudinal (A) ; ladite phase d'enveloppement étant précédée d'une synchronisation du récipient (1) et de l'étiquette (8) de manière à ce que la partie transparente (9) soit placée sur les repères gradués (4).

2. Le procédé selon la revendication 1, **caractérisé en ce que** ladite synchronisation est obtenue en faisant tourner le récipient (1) à une vitesse constante autour de son propre axe longitudinal (A), en utilisant un capteur (27) pour détecter un repère (28) sur le récipient (1), en calculant l'intervalle de temps, à partir de l'instant de détection, pendant lequel le récipient (1) doit être maintenu en rotation, à ladite vitesse constante, pour qu'il atteigne une position angulaire prédéfinie.

3. Le procédé selon la revendication 1, **caractérisé en ce que** ladite synchronisation est obtenue en faisant tourner le récipient (1) à une vitesse constante autour de son propre axe longitudinal (A), en utilisant une caméra (27) pour détecter une image du récipient (1) coïncidant avec une image échantillon, en calculant l'intervalle de temps, à partir de l'instant de détection, pendant lequel le récipient (1) doit être maintenu en rotation, à ladite vitesse constante, pour qu'il atteigne une position angulaire prédéfinie.

4. Le procédé selon l'une quelconque des revendications de 1 à 3, **caractérisé en ce que** la partie transparente (9) est une bande longitudinale de l'étiquette (8).

5. Le procédé selon la revendication 4, **caractérisé en ce que** la bande longitudinale est essentiellement rectangulaire.

6. Un récipient jetable comprenant un corps (2) transparent portant des repères gradués (4) et ayant une étiquette (8), **caractérisé en ce que** l'étiquette est entièrement enveloppée autour du corps (2) et présente une partie transparente (9) placée sur les repères gradués (4) et une partie non transparente enveloppée autour du corps (2) transparent.

7. Une seringue jetable comprenant un corps (2) transparent contenant un médicament, et un piston (3) associé de façon coulissante et étanche audit corps (2) pour distribuer le médicament, le corps (2) portant des repères gradués (4) et ayant une étiquette (8; 8") décrivant le médicament enveloppée autour de lui, la seringue (1) étant **caractérisée en ce que** l'étiquette (8; 8") est entièrement enveloppée autour du corps (2) et présente une partie transparente (9) placée sur les repères gradués (4) et une partie non transparente enveloppée autour du corps (2) transparent.

8. La seringue selon la revendication 7, **caractérisée en ce que** la partie transparente (9) est une bande longitudinale de l'étiquette (8, 8").

9. La seringue selon la revendication 8, **caractérisée en ce que** la bande longitudinale est essentiellement rectangulaire.

10. La seringue selon l'une quelconque des revendications de 7 à 9, **caractérisée en ce que** l'étiquette (8) est enveloppée autour du corps (2) de la seringue (1) sur 360°.
